# EUROPEAN PATENT SPECIFICATION

(11) **EP 1 446 118 B1**
(45) Date of publication and mention of the grant of the patent: **09.01.2008**
(21) Application number: 02789593.7
(22) Date of filing: 12.11.2002
(51) Int. Cl.: A61K 31/42, A61P 29/00

(54) **ORAL DOSAGE FORM OF PARECOXIB**
ORAL VERABREICHBARE ZUSAMMENSETZUNG ENTHALTEND PARECOXIB
FORME POSOLOGIQUE ORALE DU PARECOXIB

(30) Priority: 13.11.2001 US 350596 P
(43) Date of publication of application: 18.08.2004
(73) Proprietor: Pharmacia Corporation, Peapack, NJ 07977 (US)
(72) Inventor: KARIM, Aziz, Skokie, IL 60077 (US); NEMA, Sandeep, Grayslake, IL 60030 (US); EWING, Gary, D., Kalamazoo, MI 49009 (US)
(74) Representative: Dekker, Henrike Cornelie Christine
(86) International application number: PCT/US2002/036253
(87) International publication number: WO 2003/041705

(56) References cited:
- WO-A-01/41762
- WO-A-01/45706
- WO-A-01/91750
- WO-A-02/080912
- US-A- 5 932 598
- US-A1- 2002 028 238

## Description

### FIELD OF THE INVENTION

The present invention relates to therapeutic methods of use of prodrugs of sulfonamide drugs, including parecoxib, a prodrug for the selective cyclooxygenase-2 (COX-2) inhibitory drug valdecoxib. More particularly, the invention relates to orally deliverable dosage forms of such prodrugs for therapeutic methods of use.

### BACKGROUND OF THE INVENTION

Inhibition of cyclooxygenase (COX) enzymes is believed to be at least the primary mechanism by which nonsteroidal anti-inflammatory drugs (NSAIDs) exert their characteristic anti-inflammatory, antipyretic and analgesic effects, through inhibition of prostaglandin synthesis. Conventional NSAIDs such as ketorolac, diclofenac, naproxen and salts thereof inhibit both the constitutively expressed COX-1 and the inflammation-associated or inducible COX-2 isoforms of cyclooxygenase at therapeutic doses. Inhibition of COX-1, which produces prostaglandins that are necessary for normal cell function, appears to account for certain adverse side effects that have been associated with use of conventional NSAIDs. By contrast, selective inhibition of COX-2 without substantial inhibition of COX-1 leads to anti-inflammatory, antipyretic, analgesic and other useful therapeutic effects while minimizing or eliminating such adverse side effects. Selective COX-2 inhibitory drugs such as celecoxib and rofecoxib, first commercially available in 1999, have therefore represented a major advance in the art. These drugs are formulated in a variety of orally deliverable dosage forms.

Parecoxib, disclosed in U.S. Patent No. 5,932,598 to Talley et al., is one of a class of N-substituted water-soluble prodrugs of selective COX-2 inhibitory drugs having a sulfonamide moiety. Parecoxib converts to the substantially water-insoluble selective COX-2 inhibitory drug valdecoxib following administration to a subject. Parecoxib also converts to valdecoxib upon exposure to water, for example upon dissolution in water.

Parecoxib, having the structural formula (I) below, itself shows weak *in vitro* inhibitory activity against both COX-1 and COX-2, while valdecoxib (II) has strong inhibitory activity against COX-2 but is a weak inhibitor of COX-1.

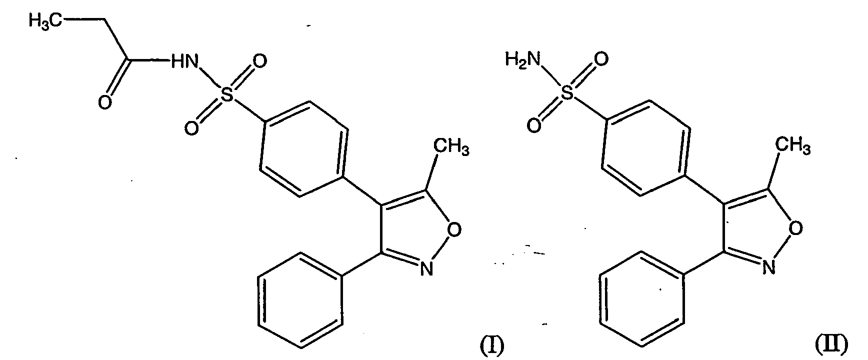

Above-cited U.S. Patent No. 5,932,598 also discloses comparably N-substituted prodrugs of other selective COX-2 inhibitors having a sulfonamide moiety. For example, the compound N-[[4-[5-(4-methylphenyl)-3-(trifluoromethyl)-1H-pyrazol-1-yl]phenyl]sulfonyl]acetamide (III) and its sodium salt are contemplated therein to be useful as prodrugs of the selective COX-2 inhibitory drug celecoxib (IV).

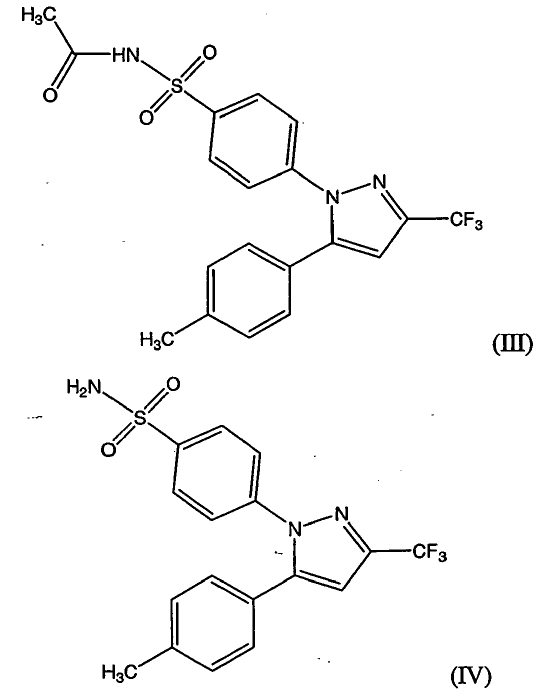

Because of the high water solubility of parecoxib, particularly of salts of parecoxib such as the sodium salt, by comparison with most selective COX-2 inhibitory drugs such as celecoxib and valdecoxib, the prodrug parecoxib has been proposed for parenteral use. See Talley et al. (2000), J. Med. Chem. 43, 1661-1663.

Above-cited U.S. Patent No. 5,932,598 indicates that a preferred method of treating inflammation is administration of the water-soluble compounds disclosed therein via injection. However, the above-cited patent further discloses that the compounds disclosed therein, or a composition comprising such a compound, may be administered orally, and that for oral administration the composition may be in the form of, for example, a tablet, hard or soft capsule, lozenge, dispensable powder, suspension or liquid.

The tendency of parecoxib to convert rapidly to insoluble valdecoxib upon exposure to water has hitherto limited any interest in oral administration of parecoxib or in developing a practical oral dosage form of parecoxib.

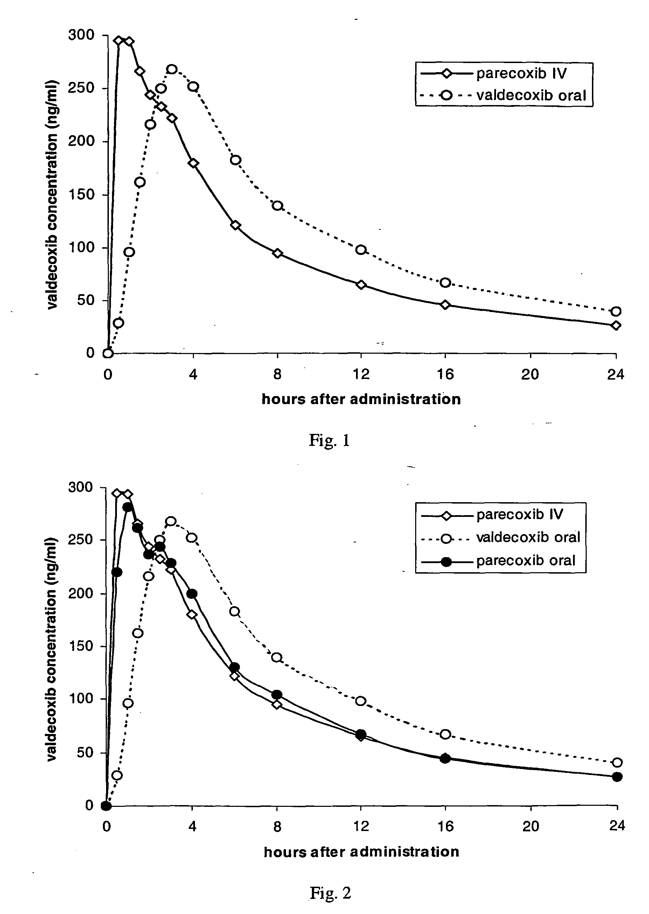

Fig. 1 shows results of a pharmacokinetic study wherein blood plasma concentration of valdecoxib was determined in 11 healthy adult subjects receiving a single intravenous (IV) 20 mg dose of parecoxib, as parecoxib sodium, in a 1 ml bolus, or a single orally administered 20 mg dose of valdecoxib in the form of an immediate release tablet, with 240 ml water. Subjects drank 180 ml water one, two and three hours postdose. Valdecoxib blood plasma concentration was determined using a validated high performance liquid chromatography (HPLC) procedure. It is clear from Fig. 1 that IV administration of parecoxib results in a maximum blood plasma concentration of valdecoxib being reached much earlier (about 1 hour after administration) than when valdecoxib itself is administered orally (about 3 hours after administration). The much more rapid establishment of a therapeutically effective concentration of valdecoxib in the bloodstream resulting from IV administration of parecoxib is important, because it can lead to significantly more rapid onset of therapeutic effect. Rapid onset is a highly desirable feature of many therapies for COX-2 mediated disorders, particularly those accompanied by acute pain.

IV administration is, for many classes of people suffering or at risk of such disorders, inconvenient and unpleasant, especially where self-administration is desired. Oral administration is generally much more convenient and conducive to a higher degree of patient compliance.

It is therefore a much desired improvement in the art of treatment and/or prophylaxis of COX-2 mediated disorders to provide, by oral administration, a time to maximum blood plasma concentration (Tₘₐₓ) of valdecoxib substantially shorter than that obtainable by oral administration of an immediate release tablet of valdecoxib, and preferably comparable in shortness of duration to that achievable by IV administration of parecoxib.

### SUMMARY OF THE INVENTION

There is now provided a method of treating or preventing a COX-2 mediated disorder in a subject, the method comprising:

Use of parecoxib as a free acid or as a pharmaceutically acceptable water-soluble salt thereof for the manufacture of composition for the treatment or prevention of a COX-2 mediated disorder in a subject,
wherein the composition is substantially free of water, is orally deliverable and has means to inhibit degradation of parecoxib to valdecoxib selected from:
● a sealed and substantially water-impermeable package or container;
● a substantially water-impermeable coating;
● a formulation of the composition having substantially no amount of any excipient that tends to promote such conversion when in intimate contact with the parecoxib or water-soluble salt thereof;
● a formulation of the composition having a barrier layer between the parecoxib or water-soluble salt thereof and an excipient that tends to promote such conversion;
and wherein the treatment consists of dissolving at least one dosage unit of the composition in a pharmaceutically acceptable aqueous vehicle to form a solution that is administered orally to the subject before substantial precipitation of insoluble matter occurs in the solution.

"Orally deliverable" herein means that the composition, either (a) as provided above, *i.e.,* substantially free of water, or (b) following dispersion and/or dissolution of the composition in a pharmaceutically acceptable aqueous vehicle, is suitable for oral administration to a subject.

It is surprisingly found that, by oral administration to a human subject of a solution derived from a composition of parecoxib or a water-soluble salt thereof as defined to for use in accordance with the invention, valdecoxib concentration in blood plasma of the subject very rapidly rises to a therapeutically effective level. Such a level is reached much more rapidly than in a comparative situation where valdecoxib itself is administered orally as an immediate release tablet formulation (Bextra® of Pharmacia Corp.). Even more surprisingly, parecoxib administered orally in accordance with the use of the invention can be comparable with, or substantially equivalent to, IV administered parecoxib in the pharmacokinetics of valdecoxib blood plasma concentration.

It is similarly found that, by oral administration to a canine subject of a solution derived from a composition of a celecoxib prodrug in accordance with the invention, celecoxib concentration in blood plasma of the subject very rapidly rises to a therapeutically effective level. Bioavailability of celecoxib from the prodrug is much greater than in a comparative situation where celecoxib itself is administered orally as a commercially available capsule formulation (Celebrex® of Pharmacia Corp.), and is similar to or greater than that obtained by oral administration of a fine celecoxib suspension in apple juice.

It is important that, in the use according to the invention where the produg is parecoxib or a water-soluble salt thereof, the solution in an aqueous vehicle be prepared within a very short period of time before administration, to minimize conversion of parecoxib to valdecoxib, which tends to occur rapidly in an aqueous medium. Such conversion can be observable by appearance of an insoluble precipitate in the solution, thus according to the use of the invention the solution is orally administered before substantial precipitation of insoluble matter occurs in the solution.

### BRIEF DESCRIPTION OF THE DRAWINGS

Fig. 1 presents data from a human pharmacokinetic study as described above, showing mean blood plasma concentrations of valdecoxib from 0 to 24 hours following (a) IV injection of 20 mg parecoxib in a 1 ml bolus; and (b) oral administration of 20 mg valdecoxib formulated as a commercial immediate release tablet.
Fig. 2 presents data from the same pharmacokinetic study, additionally showing mean blood plasma concentration of valdecoxib from 0 to 24 hours following (c) oral administration of 20 mg parecoxib as an aqueous solution.

### DETAILED DESCRIPTION OF THE INVENTION

The invention is described herein with particular reference to parecoxib, more particularly parecoxib sodium, which is a prodrug for the selective COX-2 inhibitory sulfonamide drug valdecoxib.

The sulfonamide drug for use according to the invention is a selective COX-2 inhibitory drug, valdecoxib. Prodrugs of sulfonamide drugs that have low solubility in water, and salts of such prodrugs, are especially suitable for use according to the invention, especially where such prodrugs or salts thereof are themselves water-soluble. By "low solubility in water" herein is meant having a solubility in pure water at 25°C not greater than about 10 µg/ml, preferably not greater than about 1 µg/ml.

Exposure of a parecoxib composition to moisture tends to cause significant conversion of parecoxib to valdecoxib. In such circumstances the composition remains therapeutically effective, valdecoxib being the active drug for which parecoxib is a prodrug, but the benefits according to the present invention, in particular the benefits of rapid attainment of therapeutic blood plasma concentration, and consequent rapid onset of therapeutic effect, would tend to be reduced by such exposure.

Therefore, the invention provides use of a pharmaceutical composition that is substantially free of water, *i.e.,* a dry composition. The term "substantially free of water" in the present context means that the amount of water present in the composition and available for reaction with the parecoxib is sufficiently low that the composition exhibits acceptable chemical stability of parecoxib for at least about 30 days, preferably at least about 6 months, most preferably at least about 2 years, when stored at room temperature (20-25°C) in a sealed water-impermeable container. "Acceptable chemical stability" herein means that the composition, following the defined time period (*e.g.*, about 30 days, about 6 months or about 2 years), passes a standard test for chemical purity of the therapeutic agent, in a preferred embodiment parecoxib or a water-soluble salt thereof, for example as may be required for approval by a regulatory authority. An example of such a test is the "5% total, 1% single impurity rule", whereby a preparation of a candidate drug or prodrug must contain not more than 59o total impurities, and not more than 1% of any single impurity.

Typically, a sufficiently low water content in the composition to provide acceptable chemical stability of parecoxib is less than about 5%, preferably less than about 2%, more preferably less than about 1%, by weight.

The composition for use according to the invention comprises at least one dosage unit comprising a therapeutically effective amount of parecoxib or a water-soluble salt thereof. A "dosage unit" herein means a portion of a pharmaceutical composition that contains an amount of a therapeutic agent suitable for a single oral administration to provide a therapeutic effect. Typically one dosage unit, or a small plurality (up to about 4) of dosage units, provides a sufficient amount of the agent to result in the desired effect. In this regard, when the terms "therapeutic effect", "therapeutically effective" and "therapeutic agent" are applied herein to a prodrug, for example parecoxib or a water-soluble salt thereof, it will be understood that these terms are being used in the broad sense applicable to a prodrug which is converted to a therapeutically active compound. It will further be understood in this context that "therapeutic" embraces prophylactic.

A dosage unit of a parecoxib composition for use according to the invention typically contains 1 mg to 200 mg, preferably 5 mg to 120 mg, more preferably 10 mg to 100 mg, for example 20 mg, about 40 mg or about 80 mg, parecoxib.

The term "parecoxib" is sometimes used herein to embrace salts of parecoxib, and sometimes in a stricter sense to mean the free acid form of the prodrug. The meaning will be clear from the context in which the term appears. In garecoxib salts, any pharmaceutically acceptable cation that forms a water-soluble salt of parecoxib can be used. Preferred water-soluble salts are alkali metal salts, the sodium salt (parecoxib sodium) being especially preferred.

According to the use of the present invention the dry composition has means to inhibit conversion of parecoxib to valdecoxib prior to dissolution in the aqueous vehicle. Such means can operate to inhibit the conversion in one or more of a variety of ways including those indicated immediately below. All such means, as present in association with a composition as herein provided, are embraced by the present invention.

An example of means to inhibit conversion of parecoxib to valdecoxib in a dry composition according to the use of the invention is a means to substantially prevent exposure of the composition to water, including atmospheric humidity, during storage and transport. Exposure to water can be substantially prevented, for example, by enclosing the composition in a sealed and substantially water-impermeable package or container. Alternatively or in addition, the composition can be coated with a substantially water-impermeable coating material, e.g., an ethylcellulose-based coating material. Individual solid particles or granules of the composition, or larger beads or whole tablets of the composition, can be so coated. If used, a coating should be selected to be readily degradable in the gastrointestinal tract, so that the benefits of rapid absorption of the drug or prodrug are not cancelled out by delay in release of the drug or prodrug from the ingested composition.

A further example of means to inhibit conversion of parecoxib to valdecoxib in a dry composition according to the use of the invention is to formulate the composition in such a way as to avoid or minimize contact of the parecoxib with any excipient other than water that would otherwise promote such conversion. For example, in one embodiment no such excipient is present in the composition. In another embodiment a barrier layer is present between the parecoxib and any such excipient present.

Illustratively, certain saccharides, for example mannitol, that can be useful excipients in a composition for use according to the invention, tend to promote conversion of parecoxib to valdecoxib in a dry composition where such an excipient is in intimate contact with the parecoxib. By pre-coating at least one of the excipient and the parecoxib with a material that minimizes contact between them, such conversion can be inhibited.

Other means to inhibit conversion of parecoxib to valdecoxib in a dry composition of the invention will be apparent to those of skill in the art.

The dry composition for use according to the invention is preferably substantially soluble in a pharmaceutically acceptable aqueous vehicle to form an orally deliverable solution. The term "substantially soluble" means that a dosage unit of the composition dissolves in a volume of the aqueous vehicle not greater than about 100 ml, preferably not greater than about 50 ml, with no visually observable insoluble residue, except optionally for slight cloudiness arising only from excipient ingredients of the composition or of the aqueous vehicle.

Any pharmaceutically acceptable aqueous liquid is suitable as the vehicle or medium for dissolution of the composition. Water, for example tap water or bottled water, is particularly suitable. Alternatively, sweetened, flavored and/or carbonated beverages such as sugar solutions, fruit juices, sodas, infusions (e.g., teas), extracts (e.g., beef extract, malt extract, yeast extract) *etc.* can be used.

A composition for use according to the invention can consist essentially of parecoxib or a water-soluble salt thereof, but optionally further comprises additional ingredients, for example pharmaceutically acceptable excipients. Such additional ingredients are preferably selected, and present in such amounts as, to be chemically compatible with parecoxib, in particular not to promote conversion of parecoxib to valdecoxib in substantial absence of water. If a desired excipient is found to promote such conversion, a composition containing that excipient should be formulated with a barrier layer to avoid or minimize contact between the excipient and the parecoxib as described above.

Examples of excipients that can be included in a composition for use according to the invention are excipients that facilitate preparation of the composition, for example by processes hereinafter described. Such excipients include pharmaceutically acceptable bulking agents, buffering agents, anti-caking agents, etc.

Further examples of excipients that can be included in a composition for use according to the invention are agents to enhance organoleptic properties upon dissolution of the composition. It has been found that parecoxib, specifically parecoxib sodium, has an unpleasantly bitter taste, and in a preferred embodiment there is included in the composition at least one organoleptic-enhancing agent selected from sweeteners, flavoring agents and taste modulators. Suitable sweeteners include soluble sugars such as dextrose, fructose, sucrose and mannitol, and synthetic sweeteners such as saccharin, cyclamic acid, acesulfame, aspartame, neotame and salts thereof. Suitable natural or synthetic flavoring agents can be selected from standard reference books, for example Fenaroli's Handbook of Flavor Ingredients, 3rd edition (1995). Examples of suitable natural flavors, some of which can readily be simulated with synthetic agents or combinations thereof, include almond, anise, apple, apricot, bergamot, blackberry, blackcurrant, blueberry, cacao, caramel, cherry, cinnamon, clove, coffee, coriander, cranberry, cumin, dill, eucalyptus, fennel, fig, ginger, grape, grapefruit, guava, hop, lemon, licorice, linne, malt, mandarin, molasses, nutmeg, orange, peach, pear, peppermint, raspberry, rose, spearmint, strawberry, tangerine, tea, vanilla, wintergreen, etc. Taste modulators are agents that affect a subject's perception of taste and include anesthetic agents.

Preferred excipients are those that dissolve completely in the aqueous vehicle. Accessory excipients can optionally be included to enhance dissolution of other ingredients; such accessory excipients include pharmaceutically acceptable wetting agents, cyclodextrins, *etc.*

The dry composition for use according to the invention can be in any suitable form, but is preferably in a rapidly dissolving form, for example a powder (e.g., a powder prepared by lyophilization as hereinafter described) or a rapidly disintegrating tablet. Optionally an effervescent agent, for example a bicarbonate salt such as sodium bicarbonate, can be included to accelerate dissolution and to provide organoleptic benefits of effervescence.

A powder composition for use according to the invention preferably has sufficient porosity to permit rapid dissolution of the therapeutic agent upon addition to an aqueous vehicle. A high degree of porosity is obtainable by using a lyophilization process to prepare the powder as described hereinbelow.

In an illustrative process, parecoxib sodium and a buffering agent, for example dibasic sodium phosphate heptahydrate, are dissolved in water to form an aqueous solution. Parecoxib sodium and the buffering agent are present in the solution at concentrations relative to each other consistent with the desired relative concentrations of these ingredients in the final composition. Absolute concentrations of these ingredients are not critical; however, in the interest of process efficiency it is generally preferred that the concentration of parecoxib sodium be as high as can be conveniently prepared without risking exceeding the limit of solubility. Other formulation ingredients can be added in this step if desired. Order of addition is not critical but it is strongly preferred to add the parecoxib sodium last to ensure rapid and complete dissolution, and to minimize the duration of exposure of the parecoxib to water.

The solution is metered into one or more lyophilization containers, e.g., vials. Each container receives a measured volume of solution having a desired dosage amount of parecoxib sodium. Stoppers having an opening to allow sublimation to occur are placed on the containers. The stoppered containers are then placed in a lyophilization chamber and the contents of the containers lyophilized, preferably in a three-phase cycle.

In the first phase of the lyophilization cycle, the solution in each container is frozen to a temperature below the glass transition temperature of the solution. For compositions comprising parecoxib sodium and dibasic sodium phosphate, the glass transition temperature is about -20°C. Glass transition temperature can be measured by any technique known in the art, for example by use of a freeze-drying microscope or by electrical resistance measurement. A suitable temperature for this freezing phase is typically -30°C to -60°C, for example -40°C to -50°C. Temperature is gradually lowered from room temperature to the desired freezing temperature, typically over a period of 1 to 5 hours, more typically 2 to 4 hours. The temperature is then held at the freezing temperature, typically for a period of 0.5 to 24 hours, more typically 0.75 to 3 hours.

In the freezing phase of a preferred lyophilization process, temperature is first lowered from room temperature to about -20°C fairly rapidly, e.g., over a period of 0.25 to 1 hour, more preferably 0.5 to 0.75 hour. Temperature is then lowered more gradually from -20°C to -30°C, e.g., over a period of 1 to 4 hours, more preferably 1.5 to 3 hours. Without being bound by theory, it is believed that this gradual lowering of temperature ensures that the solution is completely frozen. Temperature is then lowered fairly rapidly from about -30°C to the final freezing temperature, preferably about -40°C, *e.g.,* over a period of 0.1 to 1 hour, more preferably 0.25 to 0.5 hour. It has been found that a stepwise freezing phase as described above tends to provide a final lyophilized product that appears solid with no cracking.

In a second phase of the lyophilization cycle, freeze-drying is effected by drawing a vacuum in the lyophilization chamber. This phase is described herein as the "primary drying" phase. A vacuum of 25 to 500 *µ*m Hg (25 to 500 millitorr), preferably 50 to 300 *µ*m Hg, is generally suitable. During the primary drying phase, temperature is gradually raised, optionally in stages separated by periods when the temperature is held constant. Preferably the vacuum is maintained with a nitrogen sweep. Ice sublimates from the frozen solution during this phase, forming a partially dried cake.

In the primary drying phase of a preferred lyophilization process, temperature is first raised from the freezing temperature, e.g., -40°C, to 0°C over a period of 1 to 5 hours, preferably 2 to 4 hours, and is then held at about 0°C for a prolonged period, for example 6 to 12 hours, preferably 8 to 10 hours. Preferably a vacuum of 150 to 300 µm Hg is used during the primary drying phase.

In a third phase of the lyophilization cycle, drying is completed under vacuum. This phase is described herein as the "secondary drying" phase. Again a vacuum of 25 to 500 µm Hg, preferably 50 to 300 µm Hg, is generally suitable, preferably maintained with a nitrogen sweep. Temperature is raised during the secondary drying phase, preferably to a level above room temperature, for example about 40°C, to drive off remaining moisture and provide a powder having a moisture content of less than about 5%, preferably less than about 2%, more preferably less than about 1%, by weight.

In the secondary drying phase of a preferred lyophilization process, temperature is first raised from 0°C to 40°C over a period of 1 to 4 hours, preferably about 1.5 to 3 hours, and is then held at 40°C for 3 to 12 hours, preferably 4 to 8 hours. Preferably a vacuum of 150 to 300 µm Hg is used during the secondary drying phase. Optionally during the last part of the secondary drying phase, while temperature is being held at about 40°C, the vacuum is lowered to 25 to 75 µm Hg.

The overall lyophilization cycle time is typically 18 to 36 hours. Extending the cycle time is generally not deleterious to quality of the finished product but increases process cost. The best combination of product quality and process economics can be found by routine testing based on the information presented herein, and will vary depending on several factors, including the particular lyophilization equipment used, the containers selected, the precise composition and concentration of ingredients in the solution being lyophilized, etc. In general, however, a cycle time of 18 to 24 hours will be found suitable. In the case of parecoxib sodium compositions having dibasic sodium phosphate as the buffering agent, it has been found that shortening cycle time substantially below about 18 hours, for example to 16.5 hours, leads to increased incidence of collapse of the finished product, which in turn is not conducive to the desired rapid dissolution upon addition to an aqueous vehicle.

On completion of the lyophilization cycle, the vacuum is released and temperature is permitted to return to room temperature. The containers are then sealed to prevent reabsorption of moisture from the atmosphere.

Discrete dosage forms such as tablets and capsules suitable for oral administration of parecoxib can be prepared by methods known in the art. Methods that minimize amount and/or duration of water contact with parecoxib are preferred

### Third embodiment of the invention: a therapeutic or prophylactic method

In a third embodiment, a method of treating or preventing a COX-2 mediated disorder in a subject is provided. The method comprises (a) dissolving, in a pharmaceutically acceptable aqueous vehicle, at least one dosage unit of a pharmaceutical composition that is substantially free of water and comprises a therapeutically effective amount of parecoxib or a water-soluble salt thereof, to form a solution, and (b) orally administering the solution to the subject before substantial precipitation of insoluble matter occurs in the solution.

The aqueous vehicle can be any pharmaceutically acceptable aqueous liquid, including those indicated hereinabove. Optionally, the aqueous vehicle can contain one or more ingredients such as sweeteners or flavoring agents to counteract the unpleasant taste of the parecoxib, whether or not the dry composition comprises such ingredients.

Any convenient volume of the aqueous liquid can be used as the vehicle for oral administration of a dosage unit of the composition. Typically a volume not greater than 100 ml is preferred, and more preferably the volume is not greater than 50 ml.

Where the dry composition is in the form of a powder, for example a lyophilized powder, it is generally most convenient to add the aqueous liquid to the container in which the powder is packaged. For this purpose, it is therefore preferred that the container be large enough to accommodate a suitable volume of liquid wherein, upon opening the container, the composition can be dissolved prior to administration.

Where the dry composition is in the form of a discrete dosage form, illustratively a tablet, one or more tablets can be added to a suitable volume of aqueous liquid in a drinking vessel, wherein the composition is dissolved prior to administration.

Agitation or stirring of the container or vessel wherein dissolution occurs may be desirable to accelerate the process of dissolution. Preferred compositions of the invention require only mild or no agitation or stirring.

The resulting solution is preferably administered as soon as dissolution is complete. Delay in administration can result in precipitation of insoluble valdecoxib in the solution, thereby reducing the benefits obtainable by the method of the invention. Typically oral administration should occur less than about 15 minutes, preferably less than about 5 minutes, after preparation of the solution.

Compositions of the invention are useful in treatment and prevention of a very wide range of disorders mediated by COX-2, including but not restricted to disorders characterized by inflammation, pain and/or fever. Such compositions are especially useful as anti-inflammatory agents, such as in treatment of arthritis, with the additional benefit of having significantly less harmful side effects than compositions of conventional NSAIDs that lack selectivity for COX-2 over COX-1. In particular, compositions of the invention have reduced potential for gastrointestinal toxicity and gastrointestinal irritation, including upper gastrointestinal ulceration and bleeding, by comparison with compositions of conventional NSAIDs. Thus compositions of the invention are particularly useful as an alternative to conventional NSAIDs where such NSAIDs are contraindicated, for example in patients with peptic ulcers, gastritis, regional enteritis, ulcerative colitis, diverticulitis or with a recurrent history of gastrointestinal lesions; gastrointestinal bleeding, coagulation disorders including anemia such as hypoprothrombinemia, hemophilia or other bleeding problems; kidney disease; or in patients prior to surgery or patients taking anticoagulants.

Contemplated compositions are useful to treat a variety of arthritic disorders, including but not limited to rheumatoid arthritis, spondyloarthropathies, gouty arthritis, osteoarthritis, systemic lupus erythematosus and juvenile arthritis.

Such compositions are useful in treatment of asthma, bronchitis, menstrual cramps, preterm labor, tendonitis, bursitis, allergic neuritis, cytomegalovirus infection, apoptosis including HIV-induced apoptosis, lumbago, liver disease including hepatitis, skin-related conditions such as psoriasis, eczema, acne, bums, dermatitis and ultraviolet radiation damage including sunburn, and post-operative inflammation including that following ophthalmic surgery such as cataract surgery or refractive surgery.

Such compositions are useful to treat gastrointestinal conditions such as inflammatory bowel disease, Crohn's disease, gastritis, irritable bowel syndrome and ulcerative colitis.

Such compositions are useful in treating inflammation in such diseases as migraine headaches, periarteritis nodosa, thyroiditis, aplastic anemia, Hodgkin's disease, sclerodoma, rheumatic fever, type I diabetes, neuromuscular junction disease including myasthenia gravis, white matter disease including multiple sclerosis, sarcoidosis, nephrotic syndrome, Behcet's syndrome, polymyositis, gingivitis, nephritis, hypersensitivity, swelling occurring after injury including brain edema, myocardial ischemia, and the like.

Such compositions are useful in treatment of ophthalmic disorders, including without limitation inflammatory disorders such as endophthalmitis, episcleritis, retinitis, iriditis, cyclitis, choroiditis, keratitis, conjunctivitis and blepharitis, inflammatory disorders of more than one part of the eye, *e.g.,* retinochoroiditis, iridocyclitis, iridocyclochoroiditis (also known as uveitis), keratoconjunctivitis, blepharoconjunctivitis, *etc.;* other COX-2 mediated retinopathies including diabetic retinopathy; ocular photophobia; acute trauma of any tissue of the eye including postsurgical trauma, e.g., following cataract or corneal transplant surgery; postsurgical ocular inflammation; intraoperative miosis; corneal graft rejection; ocular, for example retinal, neovascularization including that following injury or infection; macular degeneration; cystoid macular edema; retrolental fibroplasia; neovascular glaucoma; and ocular pain.

Such compositions are useful in treatment of pulmonary inflammation, such as that associated with viral infections and cystic fibrosis, and in bone resorption such as that associated with osteoporosis.

Such compositions are useful for treatment of certain central nervous system disorders, such as cortical dementias including Alzheimer's disease, neurodegeneration, and central nervous system damage resulting from stroke, ischemia and trauma. The term "treatment" in the present context includes partial or total inhibition of dementias, including Alzheimer's disease, vascular dementia, multi-infarct dementia, pre-senile dementia, alcoholic dementia and senile dementia.

Such compositions are useful in treatment of allergic rhinitis, respiratory distress syndrome, endotoxin shock syndrome and liver disease.

Such compositions are useful in treatment of pain, including but not limited to postoperative pain, dental pain, muscular pain, and pain resulting from cancer. For example, such compositions are useful for relief of pain, fever and inflammation in a variety of conditions including rheumatic fever, influenza and other viral infections including common cold, low back and neck pain, dysmenorrhea, headache, toothache, sprains and strains, myositis, neuralgia, synovitis, arthritis, including rheumatoid arthritis, degenerative joint diseases (osteoarthritis), gout and ankylosing spondylitis, bursitis, bums, and trauma following surgical and dental procedures.

Such compositions are useful for treating and preventing inflammation-related cardiovascular disorders, including vascular diseases, coronary artery disease, aneurysm, vascular rejection, arteriosclerosis, atherosclerosis including cardiac transplant atherosclerosis, myocardial infarction, embolism, stroke, thrombosis including venous thrombosis, angina including unstable angina, coronary plaque inflammation, bacterial-induced inflammation including Chlamydia-induced inflammation, viral induced inflammation, and inflammation associated with surgical procedures such as vascular grafting including coronary artery bypass surgery, revascularization procedures including angioplasty, stent placement, endarterectomy, or other invasive procedures involving arteries, veins and capillaries.

Such compositions are useful in treatment of angiogenesis-related disorders in a subject, for example to inhibit tumor angiogenesis. Such compositions are useful in treatment of neoplasia, including metastasis; ophthalmological conditions such as corneal graft rejection, ocular neovascularization, retinal neovascularization including neovascularization following injury or infection, diabetic retinopathy, macular degeneration, retrolental fibroplasia and neovascular glaucoma; ulcerative diseases such as gastric ulcer; pathological, but non-malignant, conditions such as hemangiomas, including infantile hemangiomas, angiofibroma of the nasopharynx and avascular necrosis of bone; and disorders of the female reproductive system such as endometriosis.

Such compositions are useful in prevention and treatment of benign and malignant tumors and neoplasia including cancer, such as colorectal cancer, brain cancer, bone cancer, epithelial cell-derived neoplasia (epithelial carcinoma) such as basal cell carcinoma, adenocarcinoma, gastrointestinal cancer such as lip cancer, mouth cancer, esophageal cancer, small bowel cancer, stomach cancer, colon cancer, liver cancer, bladder cancer, pancreas cancer, ovary cancer, cervical cancer, lung cancer, breast cancer, skin cancer such as squamous cell and basal cell cancers, prostate cancer, renal cell carcinoma, and other known cancers that effect epithelial cells throughout the body. Neoplasias for which compositions of the invention are contemplated to be particularly useful are gastrointestinal cancer, Barrett's esophagus, liver cancer, bladder cancer, pancreatic cancer, ovarian cancer, prostate cancer, cervical cancer, lung cancer, breast cancer and skin cancer. Such compositions can also be used to treat fibrosis that occurs with radiation therapy. Such compositions can be used to treat subjects having adenomatous polyps, including those with familial adenomatous polyposis (FAP). Additionally, such compositions can be used to prevent polyps from forming in subjects at risk of FAP.

Such compositions inhibit prostanoid-induced smooth muscle contraction by inhibiting synthesis of contractile prostanoids and hence can be of use in treatment of dysmenorrhea, premature labor, asthma and eosinophil-related disorders. They also can be of use for decreasing bone loss particularly in postmenopausal women (i.e., treatment of osteoporosis), and for treatment of glaucoma.

Preferred uses for compositions of the present invention are for treatment of rheumatoid arthritis and osteoarthritis, for pain management generally (particularly post-oral surgery pain, post-general surgery pain, post-orthopedic surgery pain, and acute flares of osteoarthritis), for prevention and treatment of headache and migraine, for treatment of Alzheimer's disease, and for colon cancer chemoprevention.

Because of the rapid onset of therapeutic effect that can be exhibited by compositions of the invention, these compositions have particular advantages over prior orally deliverable compositions of selective COX-2 inhibitory drugs for treatment of acute COX-2 mediated disorders, especially for relief of pain, for example in headache, including sinus headache and migraine.

Besides being useful for human treatment, compositions of the invention are useful for veterinary treatment of companion animals, exotic animals, farm animals, and the like, particularly mammals. More particularly, compositions of the invention are useful for treatment of COX-2 mediated disorders in horses, dogs and cats.

The dosage regimen to prevent, give relief from, or ameliorate the condition or disorder preferably corresponds to once-a-day or twice-a-day treatment, but can be modified in accordance with a variety of factors. These include the type, age, weight, sex, diet and medical condition of the subject and the nature and severity of the disorder. Thus, the dosage regimen actually employed can vary widely and can therefore deviate from the preferred dosage regimens set forth above.

Initial treatment can begin with a dose regimen as indicated above. Treatment is generally continued as necessary over a period of several weeks to several months or years until the condition or disorder has been controlled or eliminated. Subjects undergoing treatment with a composition of the invention can be routinely monitored by any of the methods well known in the art to determine effectiveness of therapy. Continuous analysis of data from such monitoring permits modification of the treatment regimen during therapy so that optimally effective doses are administered at any point in time, and so that the duration of treatment can be determined. In this way, the treatment regimen and dosing schedule can be rationally modified over the course of therapy so that the lowest amount of the composition exhibiting satisfactory effectiveness is administered, and so that administration is continued only for so long as is necessary to successfully treat the condition or disorder.

It has been found that parecoxib, when administered orally to a human subject, is rapidly and completely converted to valdecoxib. Surprisingly, therefore, even where rapid onset of therapeutic effect is desired, a therapeutically effective dose of parecoxib, for example in the form of parecoxib sodium, is one that is equal to a therapeutically effective dose of valdecoxib administered orally. The term "equal" in this context means equal in molar amount or in absolute amount (*i.e.,* in weight). Based on molecular weights, complete conversion of 1 mg parecoxib produces about 0.85 mg valdecoxib. For practical purposes, no great error arises from considering 1 mg parecoxib to be equivalent to 1 mg valdecoxib.

Thus according to the present invention, a method is provided for treatment of a COX-2 mediated disorder in a human subject comprising orally administering parecoxib or a salt thereof to the subject at a parecoxib dosage equal to a therapeutically effective dosage of valdecoxib. Preferably, the parecoxib or salt thereof, for example the sodium salt, is administered in a daily dosage amount of 1 mg to 200 mg. More preferred daily dosage amounts are 5 mg to 120 mg, more preferably 10 mg to 100 mg, for example about 20 mg, about 40 mg or about 80 mg, parecoxib.

In an especially surprising finding, illustrated in Fig. 2, so rapid and complete is the conversion of parecoxib to valdecoxib that oral administration of parecoxib to a human subject provides a significantly earlier peak of blood plasma concentration of valdecoxib than does oral administration of valdecoxib itself at equal dose in immediate release form.

Therapeutic uses of the present invention further include combination therapies of parecoxib or a composition of the invention with one or more drugs selected from opioids and other analgesics, including narcotic analgesics, Mu receptor antagonists, Kappa receptor antagonists, non-narcotic (i.e. non-addictive) analgesics, monoamine uptake inhibitors, adenosine regulating agents, cannabinoid derivatives, Substance P antagonists, neurokinin-1 receptor antagonists and sodium channel blockers, among others. Preferred combination therapies comprise use of a composition of the invention with one or more compounds selected from aceclofenac, acemetacin, ε-acetamidocaproic acid, acetaminophen, acetaminosalol, acetanilide, acetylsalicylsalicylic acid, *S*-adenosylmethionine, alclofenac, alfentanil, allylprodine, alminoprofen, aloxiprin, alphaprodine, aluminum bis(acetylsalicylate), amfenac, aminochlorthenoxazin, 3-amino-4-hydroxybutyric acid, 2-amino-4-picoline, aminopropylon, aminopyrine, amixetrine, ammonium salicylate, ampiroxicam, amtolmetin guacil, anileridine, antipyrine, antipyrine salicylate, antrafenine, apazone, aspirin, balsalazide, bendazac, benorylate, benoxaprofen, benzpiperylon, benzydamine, benzylmorphine, berberine, bermoprofen, bezitramide, α-bisabolol, bromfenac, *p*-bromoacetanilide, 5-bromosalicylic acid acetate, bromosaligenin, bucetin, bucloxic acid, bucolome, bufexamac, bumadizon, buprenorphine, butacetin, butibufen, butorphanol, calcium acetylsalicylate, carbamazepine, carbiphene, carprofen, carsalam, chlorobutanol, chlorthenoxazin, choline salicylate, cinchophen, cinmetacin, ciramadol, clidanac, clometacin, clonitazene, clonixin, clopirac, clove, codeine, codeine methyl bromide, codeine phosphate, codeine sulfate, cropropamide, crotethamide, desomorphine, dexoxadrol, dextromoramide, dezocine, diampromide, diclofenac, difenamizole, difenpiramide, diflunisal, dihydrocodeine, dihydrocodeinone enol acetate, dihydromorphine, dihydroxyaluminum acetylsalicylate, dimenoxadol, dimepheptanol, dimethylthiambutene, dioxaphetyl butyrate, dipipanone, dipyrocetyl, dipyrone, ditazol, droxicam, emorfazone, enfenamic acid, epirizole, eptazocine, etanercept, etersalate, ethenzamide, ethoheptazine, ethoxazene, ethylmethylthiambutene, ethylmorphine, etodolac, etofenamate, etonitazene, eugenol, felbinac, fenbufen, fenclozic acid, fendosal, fenoprofen, fentanyl, fentiazac, fepradinol, feprazone, floctafenine, flufenamic acid, flunoxaprofen, fluoresone, flupirtine, fluproquazone, flurbiprofen, fosfosal, gentisic acid, glafenine, glucametacin, glycol salicylate, guaiazulene, hydrocodone, hydromorphone, hydroxypethidine, ibufenac, ibuprofen, ibuproxam, imidazole salicylate, indomethacin, indoprofen, infliximab, interleukin-10, isofezolac, isoladol, isomethadone, isonixin, isoxepac, isoxicam, ketobemidone, ketoprofen, ketorolac, *p*-lactophenetide, lefetamine, levorphanol, lexipafant, lofentanil, lonazolac, lornoxicam, loxoprofen, lysine acetylsalicylate, magnesium acetylsalicylate, meclofenamic acid, mefenamic acid, meperidine, meptazinol, mesalamine, metazocine, methadone, methotrimeprazine, metiazinic acid, metofoline, metopon, mofebutazone, mofezolac, morazone, morphine, morphine hydrochloride, morphine sulfate, morpholine salicylate, myrophine, nabumetone, nalbuphine, 1-naphthyl salicylate, naproxen, narceine, nefopam, nicomorphine, nifenazone, niflumic acid, nimesulide, 5'-nitro-2'-propoxyacetanilide, norlevorphanol, normethadone, normorphine, norpipanone, olsalazine, opium, oxaceprol, oxametacine, oxaprozin, oxycodone, oxymorphone, oxyphenbutazone, papaveretum, paranyline, parsalmide, pentazocine, perisoxal, phenacetin, phenadoxone, phenazocine, phenazopyridine hydrochloride, phenocoll, phenoperidine, phenopyrazone, phenyl acetylsalicylate, phenylbutazone, phenyl salicylate, phenyramidol, piketoprofen, piminodine, pipebuzone, piperylone, pirazolac, piritramide, piroxicam, pirprofen, pranoprofen, proglumetacin, proheptazine, promedol, propacetamol, propiram, propoxyphene, propyphenazone, proquazone, protizinic acid, ramifenazone, remifentanil, rimazolium metilsulfate, salacetamide, salicin, salicylamide, salicylamide *o*-acetic acid, salicylsulfuric acid, salsalate, salverine, simetride, sodium salicylate, sufentanil, sulfasalazine, sulindac, superoxide dismutase, suprofen, suxibuzone, talniflumate, tenidap, tenoxicam, terofenamate, tetrandrine, thiazolinobutazone, tiaprofenic acid, tiaramide, tilidine, tinoridine, tolfenamic acid, tolmetin, tramadol, tropesin, viminol, xenbucin, ximoprofen, zaltoprofen, ziconotide and zomepirac (see The Merck Index, 13th Edition (2001), Therapeutic Category and Biological Activity Index, lists therein headed "Analgesic", "Anti-inflammatory" and "Antipyretic").

Particularly preferred combination therapies comprise use of parecoxib according to the invention with an opioid compound, more particularly where the opioid compound is codeine, meperidine, morphine or a derivative thereof.

The drug being used in combination therapy with parecoxib according to the invention can be administered by any route, including parenterally, orally, topically, etc. Where both the parecoxib and the drug to be administered in combination therewith are both delivered orally, they can be formulated separately or co-formulated in a composition of the invention. Where parecoxib is co-formulated with a second drug, for example an opioid drug, the second drug can be formulated in immediate-release, rapid-onset, sustained-release or dual-release form.

In an embodiment of the invention, particularly where the COX-2 mediated condition is headache or migraine, the present parecoxib composition is administered in combination therapy with a vasomodulator, preferably a xanthine derivative having vasomodulatory effect, more preferably an alkylxanthine compound.

Combination therapies wherein an alkylxanthine compound is co-administered with a parecoxib composition as provided herein are embraced by the present embodiment of the invention whether or not the alkylxanthine is a vasomodulator and whether or not the therapeutic effectiveness of the combination is to any degree attributable to a vasomodulatory effect. The term "alkylxanthine" herein embraces xanthine derivatives having one or more C₁₋₄ alkyl, preferably methyl, substituents, and pharmaceutically acceptable salts of such xanthine derivatives. Dimethylxanthines and trimethylxanthines, including caffeine, theobromine and theophylline, are especially preferred. Most preferably, the alkylxanthine compound is caffeine.

The total and relative dosage amounts of parecoxib and of the vasomodulator or alkylxanthine are selected to be therapeutically and/or prophylactically effective for relief of pain associated with the headache or migraine. Suitable dosage amounts will depend on the particular vasomodulator or alkylxanthine selected. For example, in a combination therapy with parecoxib and caffeine, typically the parecoxib will be administered in a daily dosage amount of 10 mg to 100 mg, preferably 20 mg to 80 mg, and the caffeine in a daily dosage amount of 1 mg to 500 mg, preferably 10 mg to 400 mg, more preferably 20 mg to 300 mg.

The vasomodulator or alkylxanthine component of the combination therapy can be administered in any suitable dosage form by any suitable route, preferably orally. The vasomodulator or alkylxanthine can optionally be coformulated with parecoxib in a single oral dosage form. Thus a composition of the invention optionally comprises both parecoxib and a vasomodulator or alkylxanthine such as caffeine, in total and relative amounts consistent with the dosage amounts set out hereinabove. Alternatively, the parecoxib can be present in a dry composition suitable for dissolution in an aqueous vehicle as provided herein, and the vasomodulator or alkylxanthine can be present in the aqueous vehicle. For example, a caffeinated beverage such as tea, coffee, or a caffeinated soda or sports beverage can be used as the vehicle for dissolution of a parecoxib composition of the invention.

### EXAMPLES

The following examples illustrate an aspect of the present invention.

### Example 1

Blood plasma concentration of valdecoxib in human subjects was determined in a pharmacokinetic study in 11 healthy adult male subjects. Each subject received each of three treatments, in randomized sequence, treatments being separated by 15 days. The treatments were:
(a) a single intravenous (IV) 20 mg dose of parecoxib, as parecoxib sodium, reconstituted in 1 ml water from a lyophilized powder and administered in a bolus;
(b) a single oral 20 mg dose of valdecoxib in the form of an immediate-release valdecoxib tablet, administered with 240 ml water; and
(c) a single 20 mg dose of parecoxib, as parecoxib sodium, reconstituted in 50 ml water from a lyophilized powder and administered orally, followed by two 25 ml washes of the container.

Following each treatment, subjects drank 180 ml water one, two and three hours after the treatment.

Valdecoxib blood plasma concentration was determined using a validated high performance liquid chromatography (HPLC) procedure. The mean plasma concentration of valdecoxib from 0 to 24 hours postdose is shown in Fig. 2. The following calculated plasma pharmacokinetic parameters for valdecoxib are given in Table 1:
Cₘₐₓ: maximum concentration (ng/ml);
Tₘₐₓ: time to reach maximum concentration (hours);
T_{½}: terminal half-life of plasma concentration (hours);
AUC₀₋₄₈: area under the curve of plasma concentration from 0 to 48 hours (ng.hr/ml)- a measure of bioavailability.

**Table 1: Pharmacokinetic parameters for valdecoxib in plasma (mean ± s.d.)**

| **parameter** | **parecoxib IV** | **valdecoxib oral** | **parecoxib oral** |
|---|---|---|---|
| Cₘₐₓ (ng/ml) | 312 ± 39 | 284 ± 62 | 297 ± 69 |
| Tₘₐₓ (hours) | 1.33 ± 0.93 | 3.11 ± 0.55 | 1.22 ± 0.56 |
| T_{½} (hours) | 7.41 ± 2.29 | 7.69 ± 2.45 | 7.33 ± 2.49 |
| AUC₀₋₄₈ (ng.hr/ml) | 2555 ± 684 | 3116 ± 604 | 2590 ± 809 |

Maximum blood plasma concentration of valdecoxib was reached earlier when parecoxib was administered intravenously (Tₘₐₓ = 1.33 hours) than when valdecoxib was administered orally (Tₘₐₓ = 3.11 hours).

Surprisingly, maximum blood plasma concentration of valdecoxib, when parecoxib was administered orally in accordance with the present invention, was achieved in no longer a time (Tₘₐₓ = 1.22 hours) than when parecoxib was administered intravenously. Furthermore, the maximum valdecoxib concentration reached (Cₘₐₓ = 297 ng/ml) was similar to that achieved with either intravenous parecoxib (Cₘₐₓ = 312 ng/ml) or oral valdecoxib (Cₘₐₓ = 284 ng/ml) administration.

### Example 2

Blood plasma concentration of celecoxib in beagle dogs was determined in a pharmacokinetic study using 6 healthy adult male subjects. Each subject received each of three treatments as detailed below. Treatments (a) and (b) were administered at an earlier time, in randomized sequence, than treatment (c), but to the same dogs. The treatments were:
(a) a single oral 200 mg dose of celecoxib in the form of a Celebrex® capsule;
(b) a single oral 200 mg dose of celecoxib in the form of a freshly prepared suspension in apple juice; and
(c) a single oral dose of compound Z in aqueous solution at a concentration of 24.1 mg/ml, equivalent to 20 mg/ml celecoxib, in an amount of 10 ml.

Each treatment was administered as a bolus dose by gastric intubation, followed by 10 ml water.

Celecoxib blood plasma concentration was determined using a validated high performance liquid chromatography (HPLC) procedure. The mean plasma concentration of celecoxib from 0 to 24 hours postdose is shown in Fig. 3. Calculated plasma pharmacokinetic parameters for celecoxib are given in Table 2.

**Table 2: Pharmacokinetic parameters for celecoxib in plasma (mean ± s.d.)**

| **parameter** | **celecoxib capsule** | **celecoxib apple juice suspension** | **compound Z solution** |
|---|---|---|---|
| Cₘₐₓ (ng/ml) | 852 ± 690 | 4602 ± 1305 | 5040 ± 1298 |
| Tₘₐₓ (hours) | 1.05 ± 1.10 | 0.33 ± 0.13 | 1.83 ± 0.68 |
| AUC (ng.hr/ml) | 6792 ± 5822 | 30635 ± 16590 | 55733 ± 32451 |

## Claims

1. Use of parecoxib as a free acid or as a pharmaceutically acceptable water-soluble salt thereof for the manufacture of composition for the treatment or prevention of a COX-2 mediated disorder in a subject,
wherein the composition is substantially free of water, is orally deliverable and has means to inhibit degradation of parecoxib to valdecoxib selected from:
• a sealed and substantially water-impermeable package or container;
• a substantially water-impermeable coating;
• a formulation of the composition having substantially no amount of any excipient that tends to promote such conversion when in intimate contact with the parecoxib or water-soluble salt thereof;
• a formulation of the composition having a barrier layer between the parecoxib or water-soluble salt thereof and an excipient that tends to promote such conversion;
and wherein the treatment consists of dissolving at least one dosage unit of the composition in a pharmaceutically acceptable aqueous vehicle to form a solution that is administered orally to the subject before substantial precipitation of insoluble matter occurs in the solution.

2. The use of claim 1, wherein the amount of parecoxib or water-soluble salt thereof in each dosage unit is 1 mg to 200 mg.

3. The use of claim 1, wherein the amount of parecoxib or water-soluble salt thereof in each dosage unit is 5 mg to 120 mg.

4. The use of claim 1, wherein the amount of parecoxib or water-soluble salt thereof in each dosage unit is 10 mg to 100 mg.

5. The use of claim 1, wherein the composition is in the form of a powder.

6. The use of claim 5, wherein the powder is prepared by a process comprising lyophilization.

7. The use of claim 1, wherein the composition is in a form of a rapidly disintegrating tablet.

8. The use of claim 7, wherein the tablet is effervescent.

9. The use of claim 1, wherein the composition further comprises at least one agent to enhance an organoleptic property of the solution.

10. The use of claim 9, wherein the at least one agent to enhance an organoleptic property of the solution is selected from sweeteners, flavouring agents and taste modulators.

11. The use of claim 10, wherein the at least one agent to enhance an organoleptic property of the solution is a sweetener.

12. The use of claim 11, wherein the sweetener is a soluble sugar selected from dextrose, fructose, sucrose and mannitol.

13. The use of claim 11, wherein the sweetener is a synthetic sweetener selected from saccharin, cyclamic acid, acesulfame, aspartame, neotame and salts thereof.

## Patentansprüche

1. Verwendung von Parecoxib als freie Säure oder als pharmazeutisch verträgliches wasserlösliches Salz davon für die Herstellung einer Zusammensetzung zur Behandlung oder Prävention einer COX-2-vermittelten Störung bei einem Subjekt,
wobei die Zusammensetzung im Wesentlichen frei von Wasser ist, oral abgebbar ist und Mittel aufweist, um einen Abbau von Parecoxib in Valdecoxib zu inhibieren, ausgewählt aus:
- einer versiegelten und im Wesentlichen wasserundurchlässigen Verpackung oder einem versiegelten oder im Wesentlichen wasserundurchlässigen Behälter;
- einer im Wesentlichen wasserundurchlässigen Beschichtung;
- einer Formulierung der Zusammensetzung, die im Wesentlichen keine Menge eines Exzipiens aufweist, das die Tendenz zeigt, eine solche Umwandlung zu begünstigen, wenn es in engem Kontakt mit dem Parecoxib oder dem wasserlöslichen Salz davon ist;
- einer Formulierung der Zusammensetzung, die eine Sperrschicht zwischen dem Parecoxib oder dem wasserlöslichen Salz davon und einem Exzipiens, das die Tendenz zeigt, eine solche Umwandlung zu begünstigen, aufweist;
und wobei die Behandlung darin besteht, wenigstens eine Dosierungseinheit der Zusammensetzung in einem pharmazeutisch verträglichen wässrigen Vehikel unter Bildung einer Lösung zu lösen, die oral an das Subjekt verabreicht wird, bevor eine wesentliche Präzipitation von unlöslichem Material in der Lösung auftritt.

2. Verwendung nach Anspruch 1, wobei die Menge an Parecoxib oder wasserlöslichem Salz davon in jeder Dosierungseinheit 1 mg bis 200 mg ist.

3. Verwendung nach Anspruch 1, wobei die Menge an Parecoxib oder wasserlöslichem Salz davon in jeder Dosierungseinheit 5 mg bis 120 mg ist.

4. Verwendung nach Anspruch 1, wobei die Menge an Parecoxib oder wasserlöslichem Salz davon in jeder Dosierungseinheit 10 mg bis 100 mg ist.

5. Verwendung nach Anspruch 1, wobei die Zusammensetzung in der Form eines Pulvers ist.

6. Verwendung nach Anspruch 5, wobei das Pulver durch ein Verfahren, das eine Lyophilisierung umfasst, hergestellt ist.

7. Verwendung nach Anspruch 1, wobei die Zusammensetzung in einer Form einer schnell zerfallenden Tablette ist.

8. Verwendung nach Anspruch 7, wobei die Tablette brausend ist.

9. Verwendung nach Anspruch 1, wobei die Zusammensetzung außerdem wenigstens ein Mittel zur Verstärkung einer organoleptischen Eigenschaft der Lösung umfasst.

10. Verwendung nach Anspruch 9, wobei das wenigstens eine Mittel zur Verstärkung einer organoleptischen Eigenschaft der Lösung ausgewählt ist aus Süßungsmitteln, Aromatisierungsmitteln und Geschmacksmodulatoren.

11. Verwendung nach Anspruch 10, wobei das wenigstens eine Mittel zur Verstärkung einer organoleptischen Eigenschaft der Lösung ein Süßungsmittel ist.

12. Verwendung nach Anspruch 11, wobei das Süßungsmittel ein löslicher Zucker ist, ausgewählt aus Dextrose, Fructose, Saccharose und Mannit.

13. Verwendung nach Anspruch 11, wobei das Süßungsmittel ein synthetisches Süßungsmittel ist, ausgewählt aus Saccharin, Cyclamsäure, Acesulfam, Aspartam, Neotam und Salzen davon.

## Revendications

1. Utilisation de parécoxib sous forme d'un acide libre ou d'un de ses sels hydrosolubles pharmaceutiquement acceptables pour la production d'une composition destinée au traitement ou à la prévention d'un trouble à médiation par COX-2 chez un sujet,
dans laquelle la composition est pratiquement dépourvue d'eau, elle peut être administrée par voie orale et elle comporte un moyen pour inhiber la dégradation du parécoxib en valdécoxib, choisi entre :
• un emballage ou récipient clos hermétiquement et substantiellement imperméable à l'eau ;
• un revêtement substantiellement imperméable à l'eau ;
• une formulation de la composition ne comprenant pratiquement aucune quantité d'un quelconque excipient qui tend à favoriser cette conversion lorsqu'il est en contact intime avec le parécoxib ou un de ses sels hydrosolubles ;
• une formulation de la composition comprenant une couche de barrière entre le parécoxib ou son sel hydrosoluble et un excipient qui tend à favoriser cette conversion ;
et dans laquelle le traitement consiste en la dissolution d'au moins une unité posologique de la composition dans un véhicule aqueux pharmaceutiquement acceptable pour former une solution qui est administrée par voie orale au sujet avant que la précipitation substantielle de matière insoluble ne se produise dans la solution.

2. Utilisation suivant la revendication 1, dans laquelle la quantité de parécoxib ou de son sel hydrosoluble dans chaque unité posologique est de 1 mg à 200 mg.

3. Utilisation suivant la revendication 1, dans laquelle la quantité de parécoxib ou de son sel hydrosoluble dans chaque unité posologique est de 5 mg à 120 mg.

4. Utilisation suivant la revendication 1, dans laquelle la quantité de parécoxib ou de son sel hydrosoluble dans chaque unité posologique est de 10 mg à 100 mg.

5. Utilisation suivant la revendication 1, dans laquelle la composition est sous forme d'une poudre.

6. Utilisation suivant la revendication 5, dans laquelle la poudre est préparée par un procédé comprenant la lyophilisation.

7. Utilisation suivant la revendication 1, dans laquelle la composition est sous forme d'un comprimé à délitement rapide.

8. Utilisation suivant la revendication 7, dans laquelle le comprimé est effervescent.

9. Utilisation suivant la revendication 1, dans laquelle la composition comprend en outre au moins un agent destiné à augmenter une propriété organoleptique de la solution.

10. Utilisation suivant la revendication 9, dans laquelle ledit au moins un agent destiné à augmenter une propriété organoleptique est choisi entre des agents édulcorants, des agents aromatisants et des modulateurs de goût.

11. Utilisation suivant la revendication 10, dans laquelle ledit au moins un agent destiné à augmenter une propriété organoleptique de la solution est un agent édulcorant.

12. Utilisation suivant la revendication 11, dans laquelle l'agent édulcorant est un sucre soluble choisi entre le dextrose, le fructose, le saccharose et le mannitol.

13. Utilisation suivant la revendication 11, dans laquelle l'agent édulcorant est un agent édulcorant synthétique choisi entre la saccharine, l'acide cyclamique, l'acésulfame, l'aspartame, le néotame et leurs sels.
